Europäisches Patentamt

European Patent Office

Office européen des brevets

(11) Publication number: **0 102 419**
**B1**

(12) EUROPEAN PATENT SPECIFICATION

(45) Date of publication of patent specification: **26.02.86**

(21) Application number: **82304475.5**

(22) Date of filing: **25.08.82**

(51) Int. Cl.⁴: **A 61 K 7/30, C 11 D 3/39**

(54) A granulated perborate salt product, and process for producing the same.

(43) Date of publication of application:
**14.03.84 Bulletin 84/11**

(45) Publication of the grant of the patent:
**26.02.86 Bulletin 86/09**

(84) Designated Contracting States:
**BE DE FR GB IT NL SE**

(56) References cited:
**US-A-3 340 152**
**US-A-3 664 961**

**P.H. LIST et al.: "Hagers Handbuch der Pharmazeutischen Praxis", 4th edition, vol. 7, part A, 1971, Berlin (DE); "Springer Verlag"**

(73) Proprietor: **WARNER-LAMBERT COMPANY**
**201 Tabor Road**
**Morris Plains New Jersey 07950 (US)**

(72) Inventor: **Eoga, Anthony B.J.**
**321 Rexland Drive**
**Boonton New Jersey 07005 (US)**

(74) Representative: **Jones, Michael Raymond et al**
**HASELTINE LAKE & CO. Hazlitt House**
**28 Southampton Buildings Chancery Lane**
**London WC2A 1AT (GB)**

Courier Press, Leamington Spa, England.

## Description

The present invention relates to the preparation of granulated materials, and more particularly to the preparation of granulated perborate salts, such as anhydrous sodium perborate, as well as to the granules thus obtained.

Perborate salts such as sodium perborate monohydrate, and sodium perborate anhydrous have enjoyed broad use in the preparation of a variety of cleansing compositions, and particularly as ingredients in denture cleansers. The perborate salts are popular because of their elevated active oxygen content and temperature stability.

Generally, anhydrous sodium perborate, of the grade and texture utilized in the preparation of denture cleansing tablets, is a fluffy material of low specific weight. These properties impede the rapid and successful preparation of compressed cleanser tablets, and the tablets so prepared frequently fracture or disintegrate. Previous efforts to increase the specific weight of anhydrous sodium perborate, to make it more compressible and thereby a more manageable tableting ingredient, have failed, and prior art efforts in this regard have relied primarily upon the addition to the entire cleansing composition of one or more tableting aids such as talc, sodium benzoate, and the like. These ingredients, however, have certain drawbacks, particularly in the instance where the tablet activity is a function of its speed of disintegration in a liquid such as water. In such instance, the tableting aids tend to prolong the disintegration time of the tablet, with the result that the activity of the tablet is delayed, and the tablet is less attractive to the consumer.

A process is disclosed in U.S. Patent No. 4,115,519 to Brichard et al., for the manufacture of sodium perborate monohydrate, that purportedly results in the preparation of granules of the monohydrate possessing the desired particle size, specific weight, abrasion resistance and flowability sought for use in connection with the compaction of dental cleanser tablets. The technique disclosed by the patent, however, is complex and costly, and requires specialized apparatus to conduct a fluidized bed particle formation in contact with hydrogen peroxide. The patentees refer to prior art processes for the formation of the monohydrate salt, and indicate that those processes, as well, are complex and expensive, and frequently yield particles that are unsuitable for the present applications.

U.S. Patent No. 3664961 teaches the use of a coagglomerant for agglomerating perborates, the amount of coagglomerant being greater than 1% by weight and the coagglomerant being a detergent composition consisting of a water-soluble organic synthetic detergent and a water-soluble builder salt.

U.S. Patent No. 3,340,152, to Hotko, discloses that polyfluorocarbons may be utilized in the manufacture of tablets, as lubricants, and in amounts by weight of the tableting composition, ranging from about 1% to about 15% by weight, to supplant such known lubricants as magnesium stearate, sodium lauryl sulfate, polyethylene glycols and the like. Hotko suggests that the fluoropolymer may be added directly to the tableting mixture, in its capacity and amount as a lubricant, and purportedly has a favorable effect on the tablet-forming process. There is no disclosure in Hotko that the fluoropolymers would serve as agglomeration or compaction aids, to facilitate the preparation of granulated materials of increased and improved specific weight.

A need therefore exists for a method and associated granular product, that provides the perborate salts, and in particular, anhydrous sodium perborate, in a form that is easily and efficiently compressed in combination with other ingredients of a tablet-forming cleanser composition.

In accordance with the present invention, a method for preparing a granulated perborate salt is disclosed, which comprises forming a mixture of the perborate salt with a polymeric fluorocarbon, the mixture containing the polymeric fluorocarbon in an amount by weight of the perborate salt, of from 0.01% to 0.70%; compacting the mixture into a plurality of preforms; and comminuting the preforms under agitation to form the granulated perborate salt. The granulate may have a particle size capable of passing through a 30 mesh screen. (U.S. Standard).

Preferably, the perborate salts may be selected from sodium perborate monohydrate, and anhydrous sodium perborate, with anhydrous sodium perborate preferred. The polymeric fluorocarbon may be selected from a variety of well known non-toxic materials, with polytetrafluoroethylene preferred. The polymeric fluorocarbon may be present in an amount preferably ranging from 0.33% to 0.66% by weight of the perborate salt. The mixture of polymeric fluorocarbon and perborate salt may be compacted by passage through a roller compactor to form preforms such as flakes, or by slugging, with the preforms preferably achieving a hardness on the order of 3.1 to 3.87 kg/cm$^2$ (20 to 25 kg/in$^2$). The preforms are thereafter comminuted, by, for example, passage through an oscillating granulator, and are reduced to granules having particle sizes ranging preferably from 16 to 30 mesh, and most preferably up to 16 mesh.

The present invention also includes the granules prepared by this process, comprising the perborate salt in admixture with a polymeric fluorocarbon, the fluorocarbon being present in an amount of from 0.01% to 0.70% by weight of the perborate salt, and preferably from 0.33% to 0.66% by weight. The granules prepared by the present process have particular utility in the instance where they are included in moldable cleanser compositions, such as denture cleansers that are compressed into tablet form for commercial use. The granules of the present invention are particularly noteworthy, in that they remain cohesive and provide sufficient density and specific weight for the perborate salt, to permit efficient and rapid handling of the perborate salt, particularly in the instance where anhydrous sodium perborate is involved.

The present method is simply practised, and does not require complex processing, machinery or large amounts of energy input.

The present invention provides a method for the granulation of perborate salts to render them better suited for incorporation into moldable cleanser compositions. The method is simple and economical to practise.

The present invention provides a method that provides a granulated perborate salt product that is mechanically stable.

The present invention relates to a method for granulated perborate salts to render them more amenable to mechanical forming such as compression, when they are incorporated in moldable compositions such as cleansers pressed into tablet form. The method comprises mixing a quantity of powdered perborate salt, such as sodium perborate monohydrate or anhydrous sodium perborate, with a compression aid comprising a polymeric fluorocarbon, the polymeric fluorocarbon present in an amount by weight of the perborate salt, of from 0.01% to 0.70%, and preferably from 0.33% to 0.66%.

The polymeric fluorocarbon may be selected from a well known group of polymeric and copolymeric substances made up of carbon and fluorine, which, in addition, may contain hydrogen and/or chlorine. The fluorocarbons may include at least one fluoroolefin; for example, polytetrafluoroethylene, and copolymers of tetrafluoroethylene and hexafluoropropylene are contemplated. Also included would be polyvinylidene fluoride, a copolymer of vinylidene fluoride and hexafluoropropylene, as well as other polymeric fluorocarbons recited in U.S. Patent No. 3,340,152 to Hotko.

The fluorocarbon polymers may be utilized in the present invention, in the form of powders having particle sizes acceptable for combination with the present perborate salts, and preferably ranging up to about 150 micrometres in size. The exact particle size may vary with an average particle size of 25 to 75 micrometres commonplace. The exact particle size is not critical to the practice of the present invention.

After the mixture of the perborate salt and the polymeric fluorocarbon has been prepared, the resulting mixture is compacted to form a plurality of cohesive preforms. In particular, compaction may be conducted on a continuous or batch process, whereby the designated amount of the fluorocarbon polymer is added to a continuous stream of the perborate salt, and this stream is thereafter roller compacted in dry form to form flakes of the mixture. Alternately, formation of the preforms may be achieved by the slugging method, whereby the mixture is agglomerated by compression in, for example, a tablet slugging press, to form a plurality of slugs. The preforms, whether flakes or slugs, may be prepared to a hardness ranging from 0.775 to 4.65 kg/cm$^2$ (from 5 kg/in$^2$ to 30 kg/in$^2$) or greater, depending upon the intended use of the granules. For example, in the instance where the granulate is to be employed in a composition for compression into denture cleanser tablets, the slug may have a hardness value of from 3.1 to 4.65 kg/cm$^2$ (20 to 30 kg/in$^2$), and will successfully participate in subsequent tableting operations. The exact hardness of the granulate is not limited, however, and the foregoing range is presented for purposes of illustration only.

Subsequent to their formation, the preforms are comminuted or reduced to the granules of the present invention, by, for example, passage through a suitable oscillating granulator, or other apparatus capable of forming granules from the preforms. Thus, compactor mills, grinders and other similar well known apparatus may be utilized to reduce the particle size of the preforms to the end size desired.

The resulting granules may be prepared to a variety of particle sizes, and in particular, may be prepared to a size not exceeding that capable of passing through a 30 mesh (U.S. Standard) screen. Preferably, and in the instance where the granules of the present invention are to be added as a component to a composition to be compressed into a cleanser tablet, the particle sizes may preferably range from 16 to 30 mesh (U.S. Standard), and may preferably range from 16 to 20 mesh (U.S. Standard). The specific mesh size may naturally vary, as indicated earlier.

The particular advantage of the present invention resides in the fact that the material being granulated, i.e. the perborate salts, and specifically anhydrous sodium perborate, are extremely difficult to process as they are generally commercially available as a fluffy powder having very low specific weight. Prior art attempts to incorporate the anhydrous sodium perborate into compositions that are thereafter molded by compression, have relied upon the addition of conventional tableting aids in exceedingly large amounts, that have adversely effected the performance of the cleanser tablets. For example, granules were prepared in accordance with the teachings of the U.S. Patent to Hotko, cited earlier, which teaches the presence of the polymeric fluorocarbons in amounts ranging from 1% to 15% by weight, and preferably from 2% to 10% by weight of the composition. The tablets prepared utilizing the teachings of Hotko, were found however to exhibit undesirable delay in disintegration time, which is crucial to the commercial acceptability and cleaning effectiveness of the tablets, in the instance where denture cleanser tablets are prepared.

By contrast, the preparation of the granules in accordance with present invention, i.e. with the polymeric fluorocarbon present within a maximum amount of 0.70% by weight of the perborate salt, confers the desirable qualities of compactability, cohesion and performance that are actually improved over conventional preparations. The perborate salt granules of the present invention are therefore broadly useful in all instances where the combination of particle strength and stability, and enhanced speed of disintegration are required.

The present invention will be better understood from a consideration of the following illustrative examples.

Example I

A quantity of anhydrous sodium perborate obtained as a fluffy powder, was prepared in a container, to which was added a quantity of polytetrafluoroethylene powder identified as Grade F5A by the E.I. duPont DeNemours & Co., Inc. In this experiment, 540 grams of the perborate were mixed with 3.25 grams of the polytetrafluoroethylene. Blending was conducted for approximately 3 minutes, after which the mixture was compressed to a hardness of 2.32 kg/cm$^2$ (15 kg/in$^2$) with a tablet slugging press having 21.43 mm (27/32") tablet dies. Thereafter, the slugs were passed through an oscillating granulator having a 16 mesh U.S. Standard screen, so that granules of the mixture of anhydrous sodium perborate and polytetrafluoroethylene were obtained having a particle size of 16 mesh (U.S. Standard).

Example II

The procedure outlined in Example I was repeated, with the exception that 750.0 grams of anhydrous sodium perborate and 1.0 gram of polytetrafluoroethylene were combined. The resulting granules maintained a comparable hardness to those prepared in Example I.

Example III

A denture cleansing composition was prepared incorporating a granulate of anhydrous sodium perborate and polytetrafluoroethylene powder, prepared in accordance with the method of the present invention, as illustrated in Examples I and II. The specific ingredients of the denture cleansing composition, including the weight amounts of the respective components of the granulate, are set forth in Table I, below.

TABLE I

Denture cleansing composition

| Ingredient | Amount (grams) |
|---|---|
| Sodium perborate monohydrate | 2865.0 g |
| Trisodium phosphate | 2094.0 g |
| FD&C green #3 | 2.85 |
| FD&C blue #1 Lake Lakolene B3016 | 17.1 |
| Sodium meta silicate (Fines-Drymet) | 1419.0 |
| Sodium perborate anhydrous | 1704.0 |
| Polytetrafluoroethylene powder | 27.9 |
| Ethylenediaminetetraacetic acid tetrasodium salt dihydrate/pure | 225.0 |
| Brazilian mint, encapsulated or spray dried | 45.0 |
| Sodium perborate special powder | 90.0 |
| Detergent | 14.4 |
| Magnesium stearate USP | 1.5 |

The above cleanser composition, including the anhydrous perborate granulate, was then mixed and pressed into 2500 tablets, in a tablet press having a tablet die of 21.43 mm (27/32"). Each tablet weighed approximately 2.835 grams and had a hardness of approximately 3.1 kg/cm$^2$ (20 kg/in$^2$). The tablets were approximately 5.26 mm (0.207 inch) thick and upon testing, were found to have available oxygen of 165 mg/tablet, with a disintegration time of 90 seconds in 102 milliliters of 45°C water. The performance of these tablets was considered above average in the measured parameters, and tablets containing the perborate granulate were determined to be desirable for use in denture cleanser compositions in tablet form.

**Claims**

1. A process for preparing a granulated perborate salt, which comprises:

forming a mixture of the perborate salt with a polymeric fluorocarbon, the mixture containing the polymeric fluorocarbon in an amount by weight of the perborate salt, of from 0.01% to 0.70%.

compacting the mixture into a plurality of preforms; and

comminuting the preforms under agitation, to form the granulated perborate salt.

2. A process according to claim 1, wherein the mixture is compacted by passage through a roller compactor, and the preforms comprise flakes.

3. A process according to claim 1, wherein the mixture is compacted by slugging.

4. A process according to any preceding claim, wherein the preforms are comminuted by passage through an oscillating granulator, to a particle size passing through a 30 mesh (U.S. Standard) screen.

5. A granulated perborate salt product, suitable for inclusion in a compactible composition, the product comprising a precompressed mixture of the perborate salt and from 0.01% to 0.70% of a polymeric fluorocarbon, based on the weight of the perborate salt.

6. A product according to claim 5, having a hardness of the order of 4.65 kg/cm$^2$ (30 kg/in$^2$).

7. A product according to claim 5, having a hardness ranging from 2.32 kg/cm$^2$ (15 kg/in$^2$) to 4.65 kg/cm$^2$ (30 kg/in$^2$).

8. A product according to claim 5, having a particle size capable of passing through a 30 mesh (U.S. Standard) screen.

9. An invention according to any preceding claim, wherein the perborate salt is selected from sodium perborate monohydrate, anhydrous sodium perborate and mixtures thereof, and is preferably anhydrous sodium perborate.

10. An invention according to any preceding claim, wherein the polymeric fluorocarbon is derived from at least one fluoroolefin.

11. An invention according to any preceding claim, wherein the polymeric fluorocarbon comprises polytetrafluoroethylene.

12. An invention according to any preceding claim, wherein the polymeric fluorocarbon is present in an amount from 0.33% to 0.66% by weight of the perborate salt.

### Patentansprüche

1. Verfahren zur Vorbereitung eines körnigen Perborat-Salzes, dadurch gekennzeichnet, dass es folgende Schritte aufweist:

Bilden einer Mischung des Perborat-Salzes mit einem polymerisierten Fluorkohlenstoff, welches 0,01% bis 0,70 Gew.% des Perborat-Salzes ausmacht;

Zusammendrücken der Mischung in eine Vielzahl von Vorformen und

Zerkleinern der Vorformen bei Bewegung, um das körnige Perborat-Salz zu bilden.

2. Verfahren gemäss Anspruch 1, dadurch gekennzeichnet, dass die Mischung beim Durchlaufen eines Rollen-Zusammendrückers zusammengedrückt wird und dass die Vorformen Flocken aufweisen.

3. Verfahren gemäss Anspruch 1, dadurch gekennzeichnet, dass die Mischung durch Aufschlagen zusammengedrückt wird.

4. Verfahren gemäss einem der vorangehenden Ansprüche, dadurch gekennzeichnet, dass die Vorformen beim Durchlaufen durch ein schwingendes Schrotsieb auf eine Teilchengrösse, die durch ein 30 mesh (US Standard) Sieb kommt, verkleinert werden.

5. Körniges Perborat-Salz, geeignet für die Verwendung in einer zusammendrückbaren Zusammensetzung, dadurch gekennzeichnet, dass es eine vorgepresste Mischung von Perborat-Salz und von 0,01 bis 0,70 Gew.% eines polymerisierten Fluorkohlenstoffes, bezogen auf das Gewicht des Perborat-Salzes, enthält.

6. Salz gemäss Anspruch 5, eine Härte von ca. 4,65 kg/cm$^2$ aufweisend.

7. Salz gemäss Anspruch 5, eine Härte zwischen 2,32 kg/cm$^2$ und 4,65 kg/cm$^2$ aufweisend.

8. Salz gemäss Anspruch 5 mit einer Teilchengrösse, welche einen Durchlauf durch ein 30 mesh (US Standard) Sieb erlaubt.

9. Salz gemäss einem der vorangehenden Ansprüche, dadurch gekennzeichnet, dass das Perborat-Salz aus der Gruppe von Natrium Perborat Monohydrate, anhydriertem Natrium Perborat und Mischungen derselben ausgewählt ist und bevorzugt anhydriertes Natrium Perborat ist.

10. Erfindung gemäss einem der vorangehenden Ansprüche, dadurch gekennzeichnet, dass der polymerisierte Fluorkohlenstoff mindestens von einem Fluorolefin abgeleitet ist.

11. Erfindung gemäss einem der vorangehenden Ansprüche, dadurch gekennzeichnet, dass der polymerisierte Fluorkohlenstoff Polytetrafluorethylen enthält.

12. Erfindung gemäss einem der vorangehenden Ansprüche, dadurch gekennzeichnet, dass der polymerisierte Fluorkohlenstoff in einem Gewichtsverhältnis von 0,33% bis 0,66% im Perborat-Salz vorhanden ist.

### Revendications

1. Procédé pour préparer un sel de perborate granulé, caractérisé en ce qu'il consiste à:

former un mélange du sel de perborate avec un fluorocarbure polymérisé, le mélange contenant le fluorocarbure polymérisé en une quantité en poids du sel de perborate de 0,01% à 0,70%;

compacter le mélange en une pluralité de préformes; et

pulvériser les préformes sous agitation pour former le sel de perborate granulé.

2. Procédé selon la revendication 1, où le mélange est rendu compact par un passage dans un compacteur à rouleaux, et les préformes comprennent des flocons.

3. Procédé selon la revendication 1, où le mélange est rendu compact par compression.

4. Procédé selon l'une des revendications qui précèdent, où les préformes sont pulvérisées par un passage dans un granulateur oscillant pour avoir des particules de taille passant à travers un tamis 30 mesh (Standard U.S.).

5. Sel de perborate granulé, convenant à l'inclusion dans une composition apte au compactage, comprenant un mélange précomprimé du sel de perborate et de 0,01% à 0,70% d'un fluorocarbure polymérisé sur la base du poids de sel de perborate.

6. Sel selon la revendication 5, ayant une dureté de l'ordre de 4,65 kg/cm² (30 kg/pouce²).

7. Sel selon la revendication 5, ayant une dureté allant de 2,32 kg/cm² (15 kg/pouce²) à 4,65 kg/cm² (30 kg/pouce²).

8. Sel selon la revendication 5, ayant des particules de taille pouvant passer à travers un tamis de 30 mesh (Standard U.S.).

9. Invention selon l'une des revendications qui précèdent, où le sel de perborate est choisi parmi le perborate de sodium monohydraté, le perborate de sodium anhydre et les mélanges de ceux-ci, la préférence étant donnée au perborate de sodium anhydre.

10. Invention selon l'une des revendications qui précèdent, où le fluorocarbure polymérisé est dérivé d'au moins une fluorooléfine.

11. Invention selon l'une des revendications qui précèdent, où le fluorocarbure polymérisé comprend le polytétrafluoroéthylène.

12. Invention selon l'une des revendications qui précèdent, où le fluorocarbure polymérisé est présent en une quantité de 0,33% à 0,66% en poids du sel de perborate.